# EUROPÄISCHE PATENTSCHRIFT

(11) **EP 3 655 056 B1**
(45) Veröffentlichungstag und Bekanntmachung des Hinweises auf die Patenterteilung: **28.08.2024**
(21) Anmeldenummer: 18742469.2
(22) Anmeldetag: 16.07.2018
(51) Int. Cl.: A61M 1/14, A61M 1/34

(54) **VERFAHREN UND VORRICHTUNGEN ZUM LEEREN EINES EFFLUENTBEUTELS NACH DER BLUTBEHANDLUNG**
METHOD AND APPARATUS FOR EMPTYING AN EFFLUENT BAG AFTER BLOOD TREATMENT
PROCÉDÉ ET APPAREIL POUR VIDER UN SACHET D'EFFLUENT APRÈS LE TRAITEMENT DU SANG

(30) Priorität: 18.07.2017 DE 102017116142; 21.11.2017 DE 102017127394
(43) Veröffentlichungstag der Anmeldung: 27.05.2020
(73) Patentinhaber: Fresenius Medical Care Deutschland GmbH, 61352 Bad Homburg (DE)
(72) Erfinder: BEISSER, Nicolas, 63454 Hanau (DE); HEIDE, Alexander, 65817 Eppstein (DE); KLEWINGHAUS, Jürgen, 61440 Oberursel (DE); RICHTER, Andreas, 61276 Weilrod (DE)
(74) Vertreter: Bobbert & Partner Patentanwälte PartmbB
(86) Internationale Anmeldenummer: PCT/EP2018/069283
(87) Internationale Veröffentlichungsnummer: WO 2019/016145

(56) Entgegenhaltungen:
- EP-A1- 2 156 855
- EP-A1- 2 465 555
- DE-A1- 102006 012 087
- US-A1- 2009 187 138
- US-A1- 2010 121 246

## Beschreibung

Die vorliegende Erfindung betrifft ein Abflussschlauchsystem gemäß Anspruch 1, ein Verfahren zum Entleeren eines Effluentbeutels gemäß Anspruch 6 und eine Blutbehandlungsvorrichtung gemäß Anspruch 10.

Aus der Praxis ist die extrakorporale Blutbehandlung bekannt. Dabei wird dem Patienten Blut entnommen, das entlang eines Blutkreislaufs extrakorporal und z. B. durch einen Blutfilter geführt wird. Der Blutfilter weist eine Blutkammer, durch welche Blut geführt wird, und eine Dialysierflüssigkeitskammer, durch welche Dialysierflüssigkeit geführt wird, auf. Beide Kammern sind durch eine semi-permeable Membran voneinander getrennt. Blut und Dialysierflüssigkeit werden zumeist im Gegenstromprinzip durch den Blutfilter geleitet. Das Blut wird im Blutfilter gereinigt, die Dialysierflüssigkeit gilt bei ihrem Austritt aus dem Blutfilter als verbraucht und wird, nun als Dialysat bezeichnet, verworfen. Neben dem Dialysat umfasst das zu verwerfende Fluid auch Filtrat, welches Wasser, das dem Blut im Blutfilter entzogen wurde, umfasst. Filtrat und Dialysat werden im Folgenden einzeln oder gemeinsam vereinfacht als Effluent bezeichnet.

Das Effluent wird in der Praxis mittels einer Effluentzulaufleitung einem Effluentbeutel zugeführt und darin zunächst aufbewahrt. Nach Beendigung der Blutbehandlung, oder in Beutelleerintervallen (Intervalle, in welchen der Beutel geleert wird) während der Blutbehandlung, wird das Effluent aus dem Effluentbeutel, der über ein Waschbecken oder einen Ausguss gehalten wird, in diesen hinein verworfen.

EP 2 465 555 beschreibt ein Hämodialysegerät, das einen Effluentbeutel mit genau einer verschließbaren Öffnung umfasst, wobei die Abflussleitung zwischen dem Dialysator und dem Effluentbeutel eine Pumpe aufweist, und der Effluentbeutel mit der Abflussleitung mittels einer Mehrwegekupplung verbunden ist.

Eine Aufgabe der vorliegenden Erfindung ist es, ein Abflussschlauchsystem zum Einsatz bei der Blutbehandlung anzugeben.

Ferner sollen ein Verfahren zum Entleeren des Effluentbeutels, ein Abflussschlauchsystem und eine Blutbehandlungsvorrichtung, mit welcher das erfindungsgemäße Verfahren durchführbar ist, angegeben werden.

Die erfindungsgemäße Aufgabe wird durch ein Abflussschlauchsystem mit den Merkmalen des Anspruchs 1 gelöst. Zudem wird sie gelöst durch ein Verfahren mit den Merkmalen des Anspruchs 6 sowie durch eine Blutbehandlungsvorrichtung mit den Merkmalen des Anspruchs 10.

Alle mit dem erfindungsgemäßen Verfahren erzielbaren Vorteile lassen sich in bestimmten erfindungsgemäßen Ausführungsformen ungeschmälert auch mit den erfindungsgemäßen Vorrichtungen erzielen.

Die vorliegende Erfindung betrifft ein Abflussschlauchsystem aufweisend einen Effluentbeutel zum Aufnehmen des bei einer Blutbehandlung anfallenden Effluents, der genau eine verschließbare Effluentöffnung oder Verbindung ze einem Äußeren des Effluentbeutels aufweist, eine zum Effluentbeutel führende Effluentzulaufleitung, eine vom Effluentbeutel wegführende Effluentablaufleitung und eine Umschalteinrichtung, mittels welcher einander ausschließend entweder eine Fluidverbindung zwischen dem Inneren des Effluentbeutels und der Effluentzulaufleitung oder eine Fluidverbindung zwischen dem Effluentbeutel und der Effluentablaufleitung hergestellt werden kann.

Die vorliegende Erfindung umfasst außerdem ein Verfahren zum Entleeren eines Effluentbeutels. In diesem Verfahren wird ein erfindungsgemäßes Abflussschlauchsystem bereitgestellt.

Ferner wird eine Umschalteinrichtung derart betätigt, dass eine Fluidverbindung zwischen dem Inneren des Effluentbeutels und dem Inneren der mit der Umschalteinrichtung verbundenen Effluentablaufleitung hergestellt wird. Ergänzend kann die Umschalteinrichtung derart betätigt werden, dass eine Fluidverbindung zwischen dem Inneren des Effluentbeutels und dem Inneren der Effluentablaufleitung unterbrochen wird.

Die erfindungsgemäße Blutbehandlungsvorrichtung ist mit einer Dialysatablaufleitung, welche der hierin als Effluentzulaufleitung bezeichneten Leitung entsprechen kann und welche einem Abflussschlauchsystem Effluent zuführt, verbunden. Mittels dieser Leitung ist sie mit einem erfindungsgemäßen Abflussschlauchsystem verbunden.

Erfindungsgemäße Ausführungsformen können manche, einige oder alle der folgenden Merkmale in beliebiger Kombination aufweisen, soweit dies für den Fachmann nicht erkennbar technisch unmöglich ist. Vorteilhafte Weiterbildungen der vorliegenden Erfindung sind jeweils auch Gegenstand der Unteransprüche.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wenn hierin von einer Ausführungsform die Rede ist, so stellt dies eine erfindungsgemäße, beispielhafte Ausführungsform dar.

Das erfindungsgemäße Abflussschlauchsystem kann optional als Kit oder Set (oder Teil hiervon) verstanden werden, welches in manchen Ausführungsformen weitere Komponenten (wie eine Ladestation) aufweist, welche nicht zum Fördern von Fluiden mit dem geförderten Fluid in Kontakt stehen.

In manchen Ausführungsformen umfasst der Effluentbeutel genau eine Effluentöffnung, die sowohl als Effluenteinlassöffnung als auch als Effluentauslassöffnung dient. Sie kann die einzige Flüssigkeitsöffnung des Effluentbeutels sein, sie kann die einzige Öffnung überhaupt des Effluentbeutels sein. In einigen Ausführungsformen weist der Effluentbeutel zusätzlich eine Entlüftungsöffnung auf.

In einigen Ausführungsformen ist der Effluentbeutel mit nur einem Schlauchabschnitt, Verbinder oder nur einer Leitung verbunden.

In einigen Ausführungsformen ist die Effluentöffnung verschließbar und/oder mit einem Deckel vorgesehen.

In einigen Ausführungsformen kann der Effluentbeutel ein Behälter jeder Art sein, beispielsweise ein Container mit flexibler Außenhaut wie einer Folie, oder aus Folie, ein Container mit einer harten Außenhaut, oder aus einer harten Außenhaut, wie ein Kanister, usw.

Die Effluentzulaufleitung kann in einer Ausführungsform des Verfahrens verwendet werden, um wahlweise entweder mit der Blutbehandlungsvorrichtung verbunden zu sein, in welchem Fall sie dem Befüllen des Effluentbeutels dienen kann, oder um in der Nähe des Ausgusses angeordnet zu werden, in welchem Fall sie dem Entleeren des Effluentbeutels dient. Klemmen können vorgesehen sein, um ein ungewünschtes Austreten von Effluent aus der Blutbehandlungsvorrichtung, während die Effluentablaufleitung zum Entleeren des Effluentbeutels von der Blutbehandlungsvorrichtung gelöst ist, zu unterbinden. Diese Ausführungsform zeichnet sich durch besonders geringen baulichen Aufwand aus. Eine Stromversorgung usw. ist bei dieser Ausgestaltung vorteilhafterweise entbehrlich. Risiken für den Patienten können von der nicht vorhandenen Stromversorgung folglich auch nicht ausgehen.

Geeignete, optionale Schlauchverbinder oder Verbinderpaare können das Lösen der Effluentzulaufleitung von der Blutbehandlungsvorrichtung erleichtern. Geeignete, optionale Halter können das Halten zumindest des freien Endes der Effluentzulaufleitung in der Nähe des Ausgusses erleichtern.

Es weist der Effluentbeutel oder das Abflussschlauchsystem zusätzlich zur vom Effluentbeutel wegführenden Effluentablaufleitung eine, vorzugsweise elektrisch isolierende, Umschalteinrichtung auf, mittels welcher eine Fluidverbindung hergestellt werden kann. Diese Fluidverbindung wird mittels der Umschalteinrichtung, einander ausschließend, entweder zwischen dem Inneren des Effluentbeutels und der Effluentzulaufleitung ("erste Stellung"), oder zwischen dem Effluentbeutel und der Effluentablaufleitung ("zweite Stellung"), hergestellt.

In einem Hochspannungstest kann festgestellt werden, dass die Umschalteinrichtung elektrisch isoliert.

So kann beispielsweise mithilfe eines Hochspannungsprüfgeräts festgestellt werden, ob die am Effluentbeutel vorgesehene Umschalteinrichtung eine Prüfspannung von 1500 Volt AC gegenüber dem Inneren des Effluentbeutels isoliert. Der Effluentbeutel kann dazu mit Natriumchloridlösung (z. B. 0,9%-ig, kurz: NaCl-Lösung) gefüllt sein. Eine erste Elektrode für die Prüfspannung wird mit der NaCl-Lösung kontaktiert, eine zweite Elektrode wird mit dem Auslass der Umschalteinrichtung kontaktiert. Die Umschalteinrichtung wird beispielsweise 10-mal geöffnet und wieder geschlossen, sodass das Innere der Umschalteinrichtung und das Innere des Schlauches mit NaCl befüllt sind. Die zweite Elektrode wird beispielsweise mit der Hochspannung von 1500 Volt AC (10 sec Anstieg, 60 sec Haltedauer, 10 sec Abstieg) beaufschlagt. Dieser Vorgang kann mehrfach wiederholt werden. Im Anschluss wird die Umschalteinrichtung geöffnet und eine Kontrollprüfung durchgeführt. Die Umschalteinrichtung kann als ausreichend isolierend gelten, wenn beim Hochspannungstest kein Spannungsdurchschlag bzw. kein unzulässiger Stromfluss festgestellt wird.

In manchen Ausführungsformen weist die Umschalteinrichtung einen Drei-Wege-Hahn (alternativ als Drei-Wege-Ventil bezeichnet) auf oder besteht hieraus.

In bestimmten Ausführungsformen besteht die Umschalteinrichtung aus Glas oder Kunststoff oder weist wenigstens eines dieser Materialien auf.

In manchen Ausführungsformen ist die Umschalteinrichtung zwischen genau zwei Stellungen umschaltbar, nämlich nur zwischen der ersten Stellung und der zweiten Stellung.

In einigen erfindungsgemäßen, beispielhaften Ausführungsformen ist die Umschalteinrichtung ein Mehr-Wege-Hahn oder -Ventil mit mehr als drei Wegen oder Anschlüssen.

In bestimmten Ausführungsformen steht die Effluentablaufleitung mit wenigstens einer Pumpe oder einem Pumpenantrieb in Förderverbindung.

In manchen Ausführungsformen weist diese Pumpe oder dieser Pumpenantrieb wenigstens einen magnetisch gelagerten oder angetriebenen Pumpabschnitt, insbesondere einen Pumpkopf, auf. Dieser Pumpabschnitt oder Pumpkopf ist beispielsweise ausgestaltet als Impellerpumpkopf oder als dessen Rotor. Diese Art der Lagerung oder des Antriebs dient dem Schutz des Patienten vor einem Stromschlag.

In einigen Ausführungsformen wird der Pumpenantrieb, insbesondere einer Blutbehandlungsvorrichtung oder einer nicht zur Blutbehandlungsvorrichtung zählenden Pumpe, manuell mit dem Pumpkopf des Abflussschlauchsystems verbunden.

In manchen Ausführungsformen wird der Betrieb des Pumpenantriebs manuell begonnen und/oder beendet.

In gewissen Ausführungsformen wird die Umschalteinrichtung derart betätigt, dass eine Fluidverbindung, welche zwischen dem Inneren des Effluentbeutels und dem Inneren der Effluentablaufleitung besteht, unterbrochen wird.

In manchen Ausführungsformen umfasst die erfindungsgemäße Blutbehandlungsvorrichtung eine Steuervorrichtung, welche konfiguriert ist, das erfindungsgemäße Verfahren durchzuführen. In anderen Ausführungsformen ist dies nicht der Fall.

In gewissen Ausführungsformen ist die Blutbehandlungsvorrichtung als Hämodialysevorrichtung, Hämofiltrationsvorrichtung oder als Hämodiafiltrationsvorrichtung ausgestaltet, insbesondere als eine Vorrichtung für die chronische Nierenersatztherapie oder für die kontinuierliche Nierenersatztherapie (CRRT = continuous renal replacement therapy).

In einigen Ausführungsformen umfasst die erfindungsgemäße Blutbehandlungsvorrichtung eine Ladestation für eine Spannungsquelle für den Pumpenantrieb der Pumpe. Die Spannungsquelle kann eine Niedervolt- oder Niederstrom-Quelle sein.

In einigen Ausführungsformen umfasst das erfindungsgemäße Abflussschlauchsystem die Ladestation für eine Spannungsquelle für den Pumpenantrieb der Pumpe, beispielsweise für einen Akku.

In einigen Ausführungsformen weist das Abflussschlauchsystem wenigstens ein Rückschlagventil, z. B. stromab der optionalen Pumpe, auf. Das Rückschlagventil kann ein ungewolltes Austreten von Effluent aus der Effluentablaufleitung vorteilhaft verhindern, was der Sauberkeit und Hygiene zugutekommt .

In einigen Ausführungsformen umfasst das erfindungsgemäße Abflussschlauchsystem wenigstens ein Konnektorpaar. Konnektorpaare sind optional. Sie können vorteilhaft dazu dienen, das Zusammenstellen des verwendeten Abflussschlauchsystems aus bekannten Komponenten zu ermöglichen.

In einigen Ausführungsformen umfasst das erfindungsgemäße Abflussschlauchsystem eine Steuer- oder Regeleinrichtung oder steht hiermit in Signalverbindung, welche zum Umschalten der Umschalteinrichtung derart, dass der Effluentbeutel, optional mittels der Verbindungsleitung, mal mit der Effluentzulaufleitung ("erste Stellung") und mal mit der Effluentablaufleitung ("zweite Stellung") in Fluidkommunikation verbunden wird, konfiguriert oder programmiert ist. Bei dieser Steuer- oder Regeleinrichtung handelt es sich vorzugsweise nicht um eine Steuer- oder Regeleinrichtung der Blutbehandlungsvorrichtung.

In einigen Ausführungsformen ist eine Steuer- oder Regelvorrichtung der Blutbehandlungsvorrichtung zum Umschalten der Umschalteinrichtung derart, dass der Effluentbeutel mal mit der Effluentzulaufleitung und mal mit der Effluentablaufleitung verbunden wird, nicht konfiguriert oder programmiert.

Die Steuer- oder Regelvorrichtung der Blutbehandlungsvorrichtung und/oder die Steuer- oder Regelvorrichtung des Abflussschlauchsystems können derart programmiert sein, dass sowohl das Umschalten mittels der Umschalteinrichtung derart, dass mal die Effluentzulaufleitung und mal die Effluentablaufleitung mit dem Effluentbeutel verbunden ist, als auch das Starten und/oder das Stoppen der mit der Effluentablaufleitung verbundenen Pumpe miteinander gekoppelt oder gemeinsam, nacheinander, abhängig voneinander usw. automatisch erfolgen.

Dabei kann z. B. die Pumpe automatisch starten, wenn - manuell oder automatisch - die Effluentablaufleitung durch Umschalten zwischen den Stellungen an der Umschalteinrichtung mit dem Effluentbeutel verbunden ist oder wird (zweite Stellung). Ergänzend oder alternativ kann z. B. die Pumpe automatisch stoppen, wenn - manuell oder automatisch - die Effluentzulaufleitung mit dem Effluentbeutel verbunden ist oder wird (erste Stellung). Hierzu nötige Sensoren, Einrichtungen, usw. können vorgesehen sein.

In manchen Ausführungsformen weist die Blutbehandlungsvorrichtung eine Steuer- oder Regelvorrichtung auf. Die Steuer- oder Regelvorrichtung kann programmiert und/oder konfiguriert sein, um das erfindungsgemäße Verfahren im Zusammenwirken mit weiteren Einrichtungen, insbesondere einer Blutbehandlungsvorrichtung, auszuführen.

Das Abflussschlauchsystem kann disposable sein.

In manchen Ausführungsformen wirkt die Umschalteinrichtung elektrisch isolierend. Hierunter kann vorzugsweise verstanden werden, dass Fluide, die mittels eines Verbinders oder Anschlusses der Umschalteinrichtung, etwa des Drei-Wege-Hahns, in die Umschalteinrichtung eindringen können (wie etwa der Effluentzustrom in den Effluentbeutel hinein), und jene Flächen, welche sie dabei berühren, mit anderen Fluiden, die mittels eines anderen Verbinders oder Anschlusses der Umschalteinrichtung in letztere eindringen können (wie etwa der Effluentabstrom aus dem Effluentbeutel heraus), und jene Flächen, welche sie dabei berühren, für die Übertragung von elektrischem Strom nicht miteinander in Kontakt kommen können und/oder Strom nicht übertragen.

In manchen Ausführungsformen wird der Effluentbeutel zu seinem Entleeren oder für wenigstens einen Entleerungsvorgang nicht von seinem Platz, der Wiegeeinrichtung und/oder der Blutbehandlungsvorrichtung abgenommen oder entfernt.

Zum erfindungsgemäßen Verfahren kann zählen, dass nach einem vollständigen oder teilweisen Entleeren des Effluentbeutels die Effluentzulaufleitung wieder mit der Blutbehandlungsvorrichtung verbunden wird und/oder mittels der Umschalteinrichtung erneut eine Fluidverbindung zwischen der Effluentzulaufleitung und dem Effluentbeutel hergestellt wird, was einer Rückkehr aus der zweiten Stellung der Umschalteinrichtung in die erste Stellung entspricht.

In manchen Ausführungsformen wird das Effluent aus dem Effluentbeutel ohne Einsatz einer Pumpe entfernt, in anderen Ausführungsformen erfolgt dies unter Einsatz wenigstens einer Pumpe.

In einigen Ausführungsformen umfasst das Verfahren ein Schließen eines in der Effluentablaufleitung angeordneten Absperrelements. Dies dient dem Absperren eines Fluidstroms über das Absperrelement hinweg.

In manchen Ausführungsformen wird das Absperrelement erst dann geöffnet, wenn die Pumpe für das Effluent, die in der Effluentzulaufleitung angeordnet ist, angehalten wurde.

In einigen Ausführungsformen weist das erfindungsgemäße oder das verwendete Abflussschlauchsystem, das sich stromab des Effluentbeutels anschließt, keinen Flussteiler auf.

Das Abflussschlauchsystem kann hingegen eine Rollenpumpe aufweisen, etwa als in der Effluentablaufleitung angeordnete Pumpe. Diese Rollenpumpe kann optional mit genau einer Zuleitung und mit genau einer Ableitung verbunden sein. Damit kann die Pumpe den in sie hineinführenden Fluss offensichtlich nicht in mehrere Flüsse teilen.

In einigen Ausführungsformen weist das Abflussschlauchsystem, das sich stromab des Effluentbeutels und/oder stromab der Pumpe der Effluentablaufleitung anschließt, keinen Verbinder auf.

In einigen Ausführungsformen weist das Abflussschlauchsystem, das sich stromab des Effluentbeutels anschließt, stromauf und/oder stromab der Pumpe der Effluentablaufleitung kein Element auf, das mit einer elektrischen Steuereinrichtung verbunden wäre, etwa in Form eines elektrisch verbundenen Verbinders. Die Pumpe ist optional hiervon ausgenommen; sie kann elektrisch verbunden sein. Optional ist aber auch sie nicht elektrisch mit z. B. einer Steuervorrichtung verbunden.

In einigen Ausführungsformen ist die Umschalteinrichtung direkt oder indirekt mit der - optional einzigen - Effluentöffnung des Effluentbeutels verbunden. Ist die Umschalteinrichtung indirekt mit der - optional einzigen - Effluentöffnung des Effluentbeutels verbunden, so kann diese Fluidverbindung über die hierin offenbarte Verbindungsleitung erfolgen, vorzugweise ausschließlich über diese. Die Stellung der Umschalteinrichtung kann dabei dafür verantwortlich sein, ob Fluid nur in den Effluentbeutel hinein oder nur aus dem Effluentbeutel heraus gelangen kann, z. B. durch die Umschalteinrichtung hindurch.

Die Verbindungsleitung kann die einzige Fluidleitung in den oder aus dem Effluentbeutel sein.

Die Verbindungsleitung kann ausschließlich mit der Umschalteinrichtung und dem Effluentbeutel verbunden sein, d. h. nicht auch noch mit weiteren Leitungen, Behältern, usw. in Fluidverbindung stehen.

Die Verbindungsleitung steht in einigen Ausführungsformen nicht mit einer Kassette, etwa einer disposable Kassette, oder einem Port hiervon, in Fluidverbindung.

In einigen Ausführungsformen ist die Umschalteinrichtung zu einer Fluidsteuerung oder -bewegung ausschließlich, jeweils direkt oder indirekt, mit der - optional einzigen - Effluentöffnung des Effluentbeutels, der Effluentzulaufleitung sowie der Effluentablaufleitung verbunden.

In einigen Ausführungsformen wird die Umschalteinrichtung derart betätigt, dass eine Fluidverbindung zwischen dem Inneren des Effluentbeutels und dem Inneren der mit der Umschalteinrichtung verbundenen Effluentablaufleitung hergestellt wird. Ergänzend wird mittels der Umschalteinrichtung, z. B. zeitgleich und/oder zwangsgeführt, ein Fluidaustausch zwischen der Effluentzulaufleitung und dem Effluentbeutel unterbunden.

In einigen Ausführungsformen ist die Umschalteinrichtung keine Klemme, kein Zwei-Wege-Ventil oder kein Ventil, das den Durchfluss durch eine Leitung oder entlang nur einer Leitung, wie einen Schlauch, in nur einer Flussrichtung variieren kann.

In einigen Ausführungsformen weisen die erfindungsgemäßen Gegenstände, insbesondere die Blutbehandlungsvorrichtung und/oder das Abflussschlauchsystem, nur einen Effluentbeutel, nicht aber zwei oder mehr Effluentbeutel auf.

In einigen Ausführungsformen betreffen die erfindungsgemäßen Verfahren nur einen Effluentbeutel, nicht aber zwei oder mehr Effluentbeutel.

In einigen Ausführungsformen erfolgt das Befüllen und/oder das Entleeren des Effluentbeutels nicht mittels Steuerung oder Regelung einer Steuer- oder Regelvorrichtung.

Manche oder alle erfindungsgemäßen Ausführungsformen können einen, mehrere oder alle der oben und/oder im Folgenden genannten Vorteile aufweisen.

Ein Vorteil der vorliegenden Erfindung besteht darin, dass der erfindungsgemäße Effluentbeutel, anders als es bei herkömmlichen Auffangbeuteln der Fall ist, nicht manuell von der Maschine entfernt und über z. B. einem Ausgussbecken entleert werden muss, was, auch angesichts des Gewichts des Effluentbeutels von bis zu 10kg, eine unbeliebte und körperlich anstrengende Tätigkeit darstellt. Er kann zu seinem Entleeren vielmehr an der Wiegeeinrichtung der Behandlungsmaschine verbleiben.

Durch die vorliegende Erfindung wird vorteilhafterweise das Risiko umgangen, dass beim Ablassen des Inhalts des Effluentbeutels ein elektrisch leitender Kontakt von der Flüssigkeit zur Erde auftritt, sodass die zulässigen Patientenableitströme überschritten würden.

Ein weiterer Vorteil der vorliegenden Erfindung besteht im geringen Aufwand, der zur Implementierung der Erfindung erforderlich ist.

Ein mittels bestimmter Ausführungsformen erzielbarer Vorteil besteht darin, dass beide Aktionen, nämlich sowohl das Befüllen als auch das Entleeren des Effluentbeutels, durch Integration der hierfür zu betätigenden Bauteile in einem einzigen Bauteil miteinander gekoppelt und somit für eine gemeinsame Betätigung miteinander verbunden sind. Die Zwangskopplung trägt dazu bei, manuelle Schritte im Zusammenhang mit dem Entleeren nicht vergessen zu können. Zudem spart man sich manuelle oder automatische Schritte ein.

Die Flusswege zwischen Ausguss oder Abfluss einerseits und Dialysator oder Patient andererseits können in der vorliegenden Erfindung vorteilhafterweise elektrisch voneinander getrennt sein, was der Sicherheit des Patienten dient.

Ferner ist es von Vorteil, dass die Umschalteinrichtung vorgesehen sein kann, um manuell betätigt zu werden. Daher erfordert die vorliegende Erfindung in vielen Ausführungsformen keinen Eingriff in die Steuerung oder Regelung der Blutbehandlungsvorrichtung und erlaubt auch deshalb vorteilhaft eine kostengünstige Nachrüstung bestehender Systeme.

Im Folgenden wird die vorliegende Erfindung unter Bezugnahme auf die beigefügten Figuren rein exemplarisch beschrieben. In ihnen bezeichnen selbe Bezugszeichen gleiche oder selbe Komponenten. Es gilt:
- **Fig. 1**: zeigt in vereinfachter Darstellung eine erfindungsgemäße Blutbehandlungsvorrichtung mit einem extrakorporalen Blutkreislauf in einer ersten Ausführungsform;
- **Fig. 2**: zeigt in vereinfachter Darstellung ein erfindungsgemäßes Abflussschlauchsystem mit einem erfindungsgemäßen Effluentbeutel, während dem Effluentbeutel aus der Effluentzulaufleitung Effluent zugeführt wird;
- **Fig. 3**: zeigt in vereinfachter Darstellung ein erfindungsgemäßes Abflussschlauchsystem mit einem erfindungsgemäßen Effluentbeutel, während aus dem Effluentbeutel Effluent in die Effluentablaufleitung abgeführt wird;
- **Fig. 4**: zeigt eine weitere Ausführungsform der erfindungsgemäßen Blutbehandlungsvorrichtung mit einem erfindungsgemäßen Abflussschlauchsystem und einem erfindungsgemäßen Effluentbeutel;
- **Fig. 5**: zeigt eine wiederum weitere Ausführungsform der erfindungsgemäßen Blutbehandlungsvorrichtung mit einem weiteren erfindungsgemäßen Abflussschlauchsystem und einem erfindungsgemäßen Effluentbeutel; und
- **Fig. 6**: zeigt ein erfindungsgemäßes Verwenden eines herkömmlichen Effluentbeutels mit einem Abflussschlauchsystem.

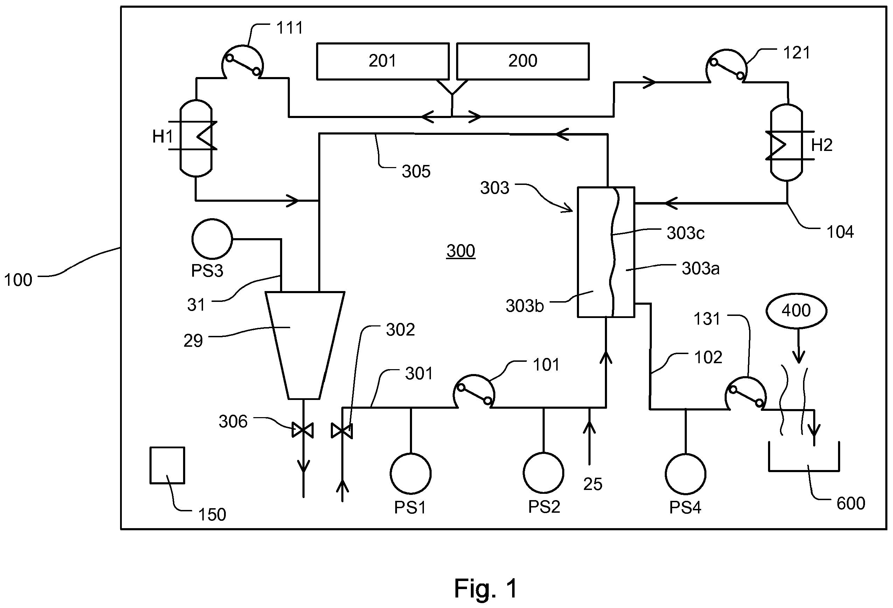

**Fig. 1** zeigt in stark vereinfachter Darstellung eine erfindungsgemäße Blutbehandlungsvorrichtung 100, verbunden mit einem extrakorporalen Blutkreislauf 300 und einem nur angedeuteten erfindungsgemäßen Abflussschlauchsystem mit einem erfindungsgemäßen Effluentbeutel 400. Abflussschlauchsystem und Effluentbeutel 400 gehen aus den folgenden Figuren hervor.

Der extrakorporale Blutkreislauf 300 weist eine erste Leitung 301, hier in Form eines arteriellen Leitungsabschnitts, auf.

Die erste Leitung 301 steht in Fluidverbindung mit einer Blutbehandlungseinrichtung, hier exemplarisch ein Blutfilter oder Dialysator 303. Der Blutfilter 303 weist eine Dialysierflüssigkeitskammer 303a und eine Blutkammer 303b auf, welche durch eine zumeist semi-permeable Membran 303c voneinander getrennt sind.

Der extrakorporale Blutkreislauf 300 weist ferner wenigstens eine zweite Leitung 305, hier in Form eines venösen Leitungsabschnitts, auf. Sowohl die erste Leitung 301 als auch die zweite Leitung 305 können zu ihrer Verbindung mit dem Gefäßsystem des nicht dargestellten Patienten dienen.

Die erste Leitung 301 ist optional mit einer (ersten) Schlauchklemme 302 zum Sperren oder Schließen der Leitung 301 verbunden. Die zweite Leitung 305 ist optional mit einer (zweiten) Schlauchklemme 306 zum Sperren oder Schließen der Leitung 305 verbunden.

Die in Fig. 1 nur durch einige ihrer Einrichtungen und schematisch repräsentierte Blutbehandlungsvorrichtung 100 weist eine Blutpumpe 101 auf. Die Blutpumpe 101 fördert während der Behandlung des Patienten Blut durch Abschnitte des extrakorporalen Blutkreislaufs 300 und in Richtung zum Blutfilter oder Dialysator 303, wie die kleinen Pfeilspitzen, welche in jeder der Figuren allgemein die Strömungsrichtung angeben, zeigen.

Mittels einer Pumpe für Dialysierflüssigkeit 121, die als Rollenpumpe oder als anderweitig okkludierende Pumpe ausgestaltet sein kann, wird frische Dialysierflüssigkeit aus einer Quelle 200 entlang der Dialysierflüssigkeitszulaufleitung 104 in die Dialysierflüssigkeitskammer 303a gepumpt. Die Dialysierflüssigkeit verlässt die Dialysierflüssigkeitskammer 303a als Dialysat, ggf. angereichert durch Filtrat, in Richtung des Ausgusses 600 und wird hierin als Effluent bezeichnet.

Die Quelle 200 kann beispielsweise ein Beutel oder ein Container sein. Die Quelle 200 kann ferner eine Fluidleitung sein, aus der online und/oder kontinuierlich erzeugte oder gemischte Flüssigkeit bereitgestellt wird, z. B. ein Hydraulikausgang oder -anschluss der Blutbehandlungsvorrichtung 100.

Eine weitere Quelle 201 mit Substituat kann optional vorgesehen sein. Sie kann der Quelle 200 entsprechen oder eine eigene Quelle sein.

Eine nur angedeutete Steuer- oder Regelvorrichtung 150 kann dazu konfiguriert sein, das vorstehende Verfahren auszuführen. Optional erfolgt eine manuelle Ausführung.

Rechts unten ist in Fig. 1 angedeutet, wo das Abflussschlauchsystem mit dem Effluentbeutel 400 mit der Blutbehandlungsvorrichtung 100 verbunden ist. Abflussschlauchsystem, Effluentbeutel 400 und Verbindung sind erst in den folgenden Figuren gezeigt.

Neben der vorgenannten Blutpumpe 101 weist die in Fig. 1 gezeigte Anordnung ferner rein optional eine Reihe weiterer, jeweils optionaler Pumpen, nämlich die Pumpe 111 für Substituat, die Pumpe 121 für Dialysierflüssigkeit und die Pumpe 131 für das Effluent, auf.

Die Pumpe 121 ist vorgesehen, um Dialysierflüssigkeit aus einer Quelle 200, beispielsweise einem Beutel, heraus und über eine optional vorhandene Beutelheizung H2 mit einem Beutel dem Blutfilter 303 mittels einer Dialysierflüssigkeitszulaufleitung 104 zuzuführen.

Die auf diese Weise zugeführte Dialysierflüssigkeit tritt über eine Dialysatablaufleitung 102, unterstützt durch die Pumpe 131, wieder aus dem Blutfilter 303 und kann verworfen werden.

Stromauf der Blutpumpe 101 ist ein optionaler arterieller Sensor PS1 vorgesehen. Während einer Behandlung des Patienten misst er den Druck in der arteriellen Leitung.

Stromab der Blutpumpe 101, jedoch stromauf des Blutfilters 303 und, falls vorgesehen, einer Zugabestelle 25 für Heparin, ist ein weiterer, optionaler Drucksensor PS2 vorgesehen. Er misst den Druck stromauf des Blutfilters 303 ("prä-Hämofilter").

Ein wiederum weiterer Drucksensor kann als PS4 stromab des Blutfilters 303, jedoch vorzugsweise stromauf der Pumpe 131, in der Dialysatablaufleitung 102 zum Messen des Filtratdrucks des Blutfilters 303 vorgesehen sein.

Blut, das den Blutfilter 303 verlässt, durchströmt eine optionale venöse Blutkammer 29, welche eine Entlüftungseinrichtung 31 aufweisen und mit einem weiteren Drucksensor PS3 in Fluidverbindung stehen kann.

Die in Fig. 1 gezeigte exemplarische Anordnung weist eine Steuer- oder Regelvorrichtung 150 auf. Sie kann mit jeder der hierin genannten Komponenten - jedenfalls oder insbesondere mit der Blutpumpe 101 - in kabelgebundener oder kabelloser Signalverbindung zur Steuerung oder Regelung der Blutbehandlungsvorrichtung 100 stehen. Sie ist optional konfiguriert, um das hierin beschriebene Verfahren auszuführen.

Die optionale Pumpe 111 ist vorgesehen, um Substituat aus der optionalen Quelle 201, beispielsweise einem Beutel, heraus und über eine optional vorhandene Beutelheizung H1 mit einem Beutel der zweiten Leitung 305 zuzuführen.

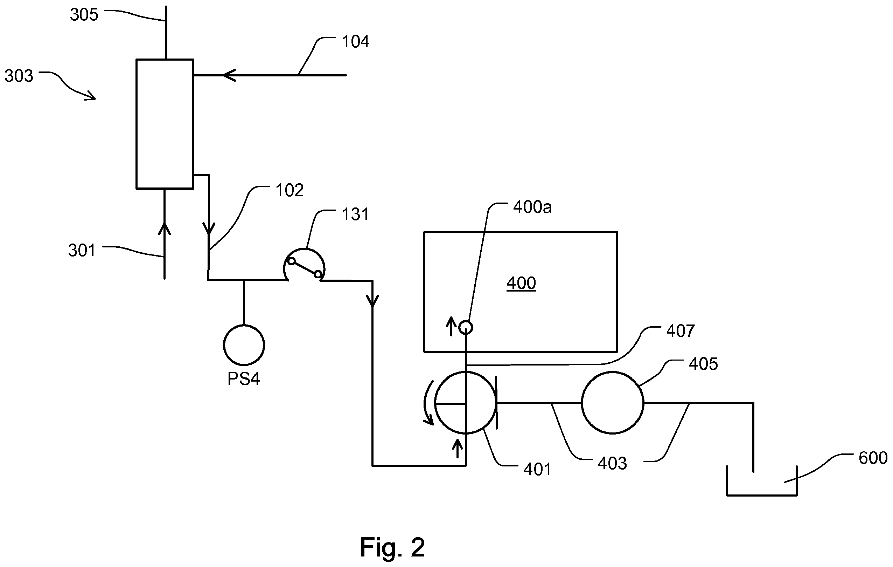

**Fig. 2** zeigt in vereinfachter Darstellung ein erfindungsgemäßes Abflussschlauchsystem mit einem erfindungsgemäßen Effluentbeutel 400 in einem Moment, während welchem dem Effluentbeutel 400 Effluent zugeführt wird.

Eine Umschalteinrichtung 401, hier exemplarisch ausgestaltet als Drei-Wege-Hahn 401, ist zwischen der Pumpe 131 für das Effluent und dem Effluentbeutel 400, jedenfalls aber stromauf zu diesem, in der Dialysatablaufleitung 102 angeordnet. Über die Dialysatablaufleitung 102 läuft Dialysat (und/oder Effluent) aus dem Blutfilter 303 ab. Sie dient zugleich aber auch als Effluentzulaufleitung, da sie dem Effluentbeutel 400 Effluent zuleitet.

Wie in Fig. 2 gezeigt ist, verbindet der Drei-Wege-Hahn 401 in seiner in Fig. 2 gezeigten Stellung die Dialysatablaufleitung 102 mit der Effluenteinlassöffnung (welche auch die Effluentauslassöffnung ist, daher kurz: Effluentöffnung 400a) des Effluentbeutels 400 fluidisch.

Gesperrt ist in dieser ersten Stellung die ebenfalls mit dem Drei-Wege-Hahn 401 verbundene Effluentablaufleitung 403, welche ihrerseits, direkt oder indirekt, mit dem Ausguss 600 verbunden ist.

In der in Fig. 2 gezeigten ersten Stellung kann Effluent aus der Dialysatablaufleitung 102 über den Drei-Wege-Hahn 401 hinweg in den Effluentbeutel 400 gelangen, nicht jedoch in die Effluentablaufleitung 403. Der Drei-Wege-Hahn 401 kann aufgrund seiner Ausgestaltung aus elektrisch isolierendem Material sein und/oder an den Stellen, an welchen er flüssigkeitsführend ist, eine elektrische Isolierung bewirken.

Die Effluentablaufleitung 403 kann eine Pumpe 405 aufweisen und in den Ausguss 600 münden.

Die Pumpe 405 ist stromab des Effluentbeutels 400, aber stromauf des Ausgusses 600, angeordnet.

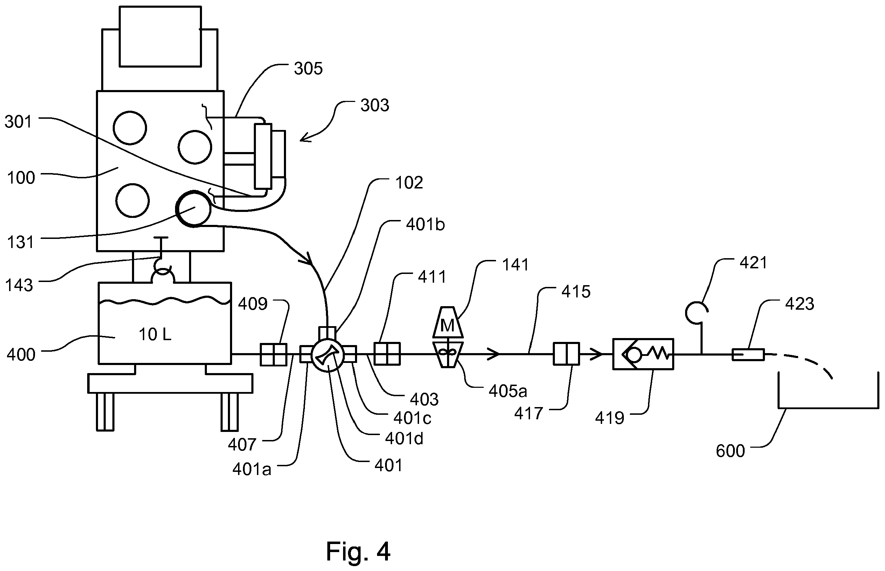

Die Pumpe 405, welche, wie erst in Fig. 4 gezeigt, optional wenigstens einen Pumpenantrieb 141 und einen Pumpkopf 405a aufweist oder hieraus bestehen kann (nicht in Fig. 2 gezeigt), ist in der Stellung der Fig. 2 nicht in Betrieb ("OFF"), da die Effluentablaufleitung 403 kein Effluent führt, welche mittels der Pumpe 405 in den Ausguss 600 verworfen werden könnte.

Die Pumpe 405 kann in jeder Ausführungsform eine Rollenpumpe sein. Alternativ ist sie in beliebigen Ausführungsformen keine Rollenpumpe.

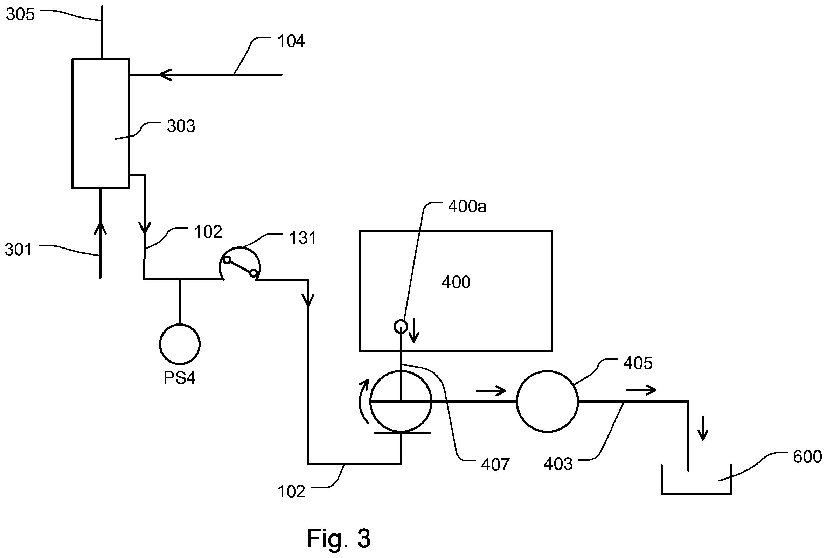

**Fig. 3** zeigt in vereinfachter Darstellung ein erfindungsgemäßes Abflussschlauchsystem mit einem erfindungsgemäßen Effluentbeutel 400, während aus dem Effluentbeutel 400 Effluent abgeführt wird.

Der Drei-Wege-Hahn 401 verbindet in seiner in Fig. 3 gezeigten Stellung die Dialysatablaufleitung 102 nicht mit der Effluentöffnung 400a des Effluentbeutels 400 fluidisch.

Gesperrt gegenüber der Dialysatablaufleitung 102 ist in dieser zweiten Stellung die ebenfalls mit dem Drei-Wege-Hahn 401 verbundene Effluentablaufleitung 403, welche ihrerseits mit dem Ausguss 600 verbunden ist.

In der in Fig. 3 gezeigten zweiten Stellung kann Effluent aus der Dialysatablaufleitung 102 nicht durch den Drei-Wege-Hahn 401 hindurch in den Ausguss 600 gelangen, und auch nicht in den Effluentbeutel 400. Die Flusswege sind dabei vorzugsweise elektrisch isoliert.

Die Pumpe 405 ist in Betrieb ("ON") und fördert Effluent aus dem Effluentbeutel 400 in den Ausguss 600.

**Fig. 4** zeigt die erfindungsgemäße Blutbehandlungsvorrichtung 100 mit einem erfindungsgemäßen, optional disposable, Abflussschlauchsystem und einem erfindungsgemäßen Effluentbeutel 400 mit weiteren, optionalen Komponenten des Abflussschlauchsystems.

Zu diesen weiteren, optionalen Komponenten zählen die Verbinder 401a, 401b und 401c, mit welchen der Drei-Wege-Hahn 401 mit einer Verbindungsleitung 407 als einem ersten Schlauchabschnitt, der Dialysatablaufleitung 102 als einem zweiten Schlauchabschnitt bzw. der Effluentablaufleitung 403 als einem dritten Schlauchabschnitt verbunden ist.

Der erste Schlauchabschnitt ist optional. Der Drei-Wege-Hahn 401 kann alternativ direkt mit der Effluentöffnung 400a verbunden sein.

Der dritte Schlauchabschnitt ist als Effluentablaufleitung 403 mit der Saugseite eines Pumpkopfs 405a einer Pumpe 405 verbunden. Der Pumpkopf 405a kann Teil der Effluentablaufleitung 403 sein, die ein Einweg-Schlauch sein kann. Der Pumpkopf 405a kann magnetisch gelagert sein, was sein Verbinden mit weiteren Abschnitten der Pumpe 405 vereinfachen kann und eine Übertragung von Strom zwischen den verbundenen Bauteilen vorteilhaft zu vermeiden hilft.

Die Druckseite des Pumpkopfs 405a ist mit einer Leitung 415 verbunden, die als eine Fortsetzung der Effluentablaufleitung 403 oder aber als vierter Schlauchabschnitt verstanden werden kann.

Vorzugsweise stromab des Pumpkopfs 405a weist die Effluentablaufleitung 403 oder die Leitung 415 ein Fixierungselement oder eine Befestigungsvorrichtung 421 auf, mittels welcher die Leitung oder das Abflussschlauchsystem insgesamt lösbar oberhalb eines Ausgusses 600 befestigt werden kann. Die Befestigungsvorrichtung 421 kann ein Haken, ein Saugnapf, usw. sein oder aufweisen.

Mittels der Befestigungsvorrichtung 421 kann die Leitung 415 oder das Abflussschlauchsystem z. B. an einem Waschbeckenrand, einem Ausguss oder dgl. befestigt werden.

Ein optionales Endstück 423 der Leitung 415 oder des Abflussschlauchsystems kann ausgestaltet sein, um z. B. einen spritzfreien Fluss oder gar Freifluss in den Ausguss 600, z. B. den Abwasserkanal der Klinik, das Waschbecken, den Kanalisationsanschluss, usw. zu gewähren. Er kann optional einen entsprechenden Verbinder, Aufsatz oder ein Gewinde usw. aufweisen.

Der Drei-Wege-Hahn 401 weist einen optionalen Griffabschnitt 401d auf, mittels welchem er wenigstens zwischen seinen oben mit Bezug auf die Fig. 2 und 3 diskutierten Stellungen bewegt werden kann. Die hierzu vorgenommenen Bewegungen sind in den Figuren 2 und 3 mittels geschwungener Pfeile angedeutet.

Verbinder 409, 411 und/oder 417 sind optional vorgesehen. Dasselbe gilt für ein vorzugsweise stromab der Pumpe 405 vorgesehenes, optionales Rückschlagventil 419.

Der Pumpenantrieb 141 der Pumpe 405 kann Teil der Behandlungsvorrichtung sein. Optional ist er hingegen Teil einer, z. B. mobilen, Vorrichtung. Letztere hat vorzugsweise keinen elektrischen und/oder körperlichen Kontakt mit der Behandlungsvorrichtung. Vorzugsweise wird die Vorrichtung von einer, vorzugsweise ladbaren, Spannungsquelle gespeist, die beim Betrieb der Pumpe nicht mit dem Stromnetz der Behandlungsvorrichtung und/oder der Klinik verbunden ist.

Der Effluentbeutel 400 ist mit einer Wiegeeinrichtung zum Wiegen seines Gewichts oder des in ihm aufgenommenen Fluids oder zum Bestimmen einer Gewichtsänderung verbunden. Beispielsweise kann der Effluentbeutel 400 als Auffangbeutel auf einer Wiegefläche der Wiegeeinrichtung liegen oder an einem Wiegehaken 143 hängen.

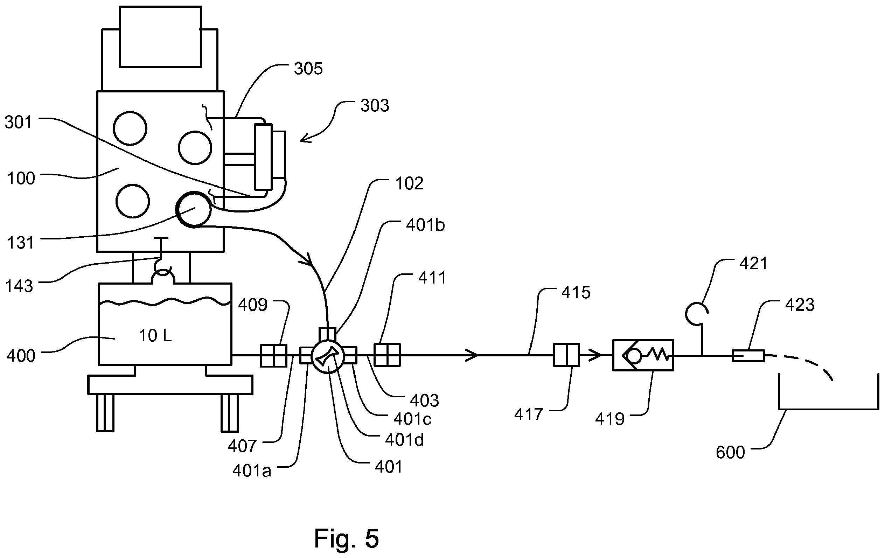

**Fig. 5** zeigt eine wiederum weitere Ausführungsform der erfindungsgemäßen Blutbehandlungsvorrichtung 100 mit einem weiteren erfindungsgemäßen Abflussschlauchsystem und einem erfindungsgemäßen Effluentbeutel 400, angelehnt an die Ausführungsform der Fig. 4.

Wie Fig. 5 zu entnehmen ist, weist das Abflussschlauchsystem keine Pumpe 405 (siehe Fig. 4) auf. Das Effluent entleert sich in der zweiten Stellung des Drei-Wege-Hahns 401 ohne Pumpenunterstützung, z. B. allein mittels Schwerkraft.

Das Rückschlagventil 419 ist auf die in dieser Ausgestaltung herrschenden Druckverhältnisse angepasst. Alternativ ist kein Rückschlagventil vorgesehen, gleich in welcher Ausführungsform.

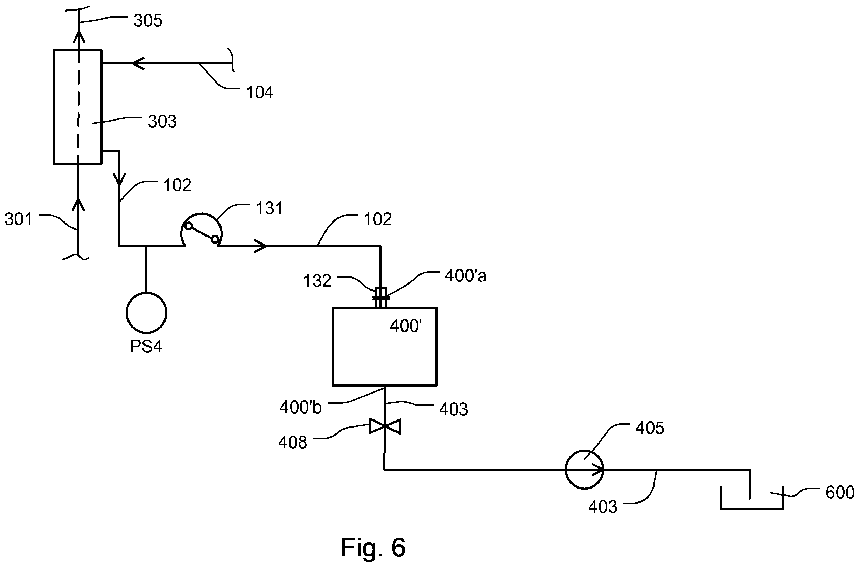

**Fig. 6** zeigt ein erfindungsgemäßes Verwenden eines herkömmlichen Effluentbeutels 400' mit einem Abflussschlauchsystem, während dem Effluentbeutel 400' Effluent zugeführt wird.

Während eines herkömmlichen Beutelwechselintervalls der Blutbehandlungsmaschine, insbesondere aus der Gruppe Dialysemaschine, Hämodiafiltrationsmaschine, Hämofiltrationsmaschine, wird der herkömmliche Effluentbeutel 400' vom Nutzer manuell zu einem Ausguss 600 getragen und über diesem entleert.

Der herkömmliche Effluentbeutel, in Fig. 6 mit dem Bezugszeichen 400' bezeichnet, weist zwei Effluent-Öffnungen auf, nämlich die bekannte Effluenteinlassöffnung 400'a mit Anschluss an der Effluentzulaufleitung 102 einerseits und die bekannte Effluentauslassöffnung 400'b mit einem, insbesondere manuellen, optionalen Absperrhahn bzw. Absperrventil 408, angeordnet in der Effluentablaufleitung 403, andererseits.

Zum Abpumpen des Effluentbeutels 400' wird während des Beutelwechselintervalls die Pumpe 131 für das Effluent angehalten. Dadurch wird der stromaufwärts der Pumpe 131 verlaufende, flüssigkeitsgefüllte Abschnitt der Effluentzulaufleitung 102 von dem stromabwärts der Pumpe 131 verlaufenden Abschnitt der flüssigkeitsgefüllten Effluentzulaufleitung 102 elektrisch isoliert. Hierdurch werden die zulässigen Grenzen der Patientenableitströme unterschritten. Die stehende, okkludierende Pumpe 131 (Rollenpumpe) isoliert dabei die stromauf der Pumpe 131 bzw. stromab der Pumpe 131 vorliegenden Flüssigkeitssäulen voneinander. Die herrschende Schwerkraft mag ein Übriges hierzu beitragen, indem die stromab der Pumpe 131 vorliegende Flüssigkeitssäule schwerkraftbedingt in Richtung des Effluentbeutels 400' abreißt oder sich in dessen Richtung bewegt.

Um die zulässigen Grenzen der Patientenableitströme weiter zu unterschreiten, kann alternativ oder zusätzlich die Effluentzulaufleitung 102 vom Effluentbeutel 400' getrennt werden. Dies kann mittels eines optional vorgesehenen Konnektors 132, der den stromabwärts der Pumpe 131 verlaufenden Abschnitt der flüssigkeitsgefüllten Effluentzulaufleitung 102 mit dem Effluentbeutel 400' verbindet, erfolgen, oder mit einer anderen Einrichtung.

Die Effluentzulaufleitung 102 kann nach ihrer Trennung optional mit einer Kappe verschlossen werden und/oder mittels einer manuellen Schlauchklemme (Kappe und Schlauchklemme sind in Figur 6 nicht gezeigt) stromabwärts der Pumpe 131 geschlossen werden.

Das optionale Öffnen des Konnektors 132 bedeutet keinen Mehraufwand gegenüber der o. g. herkömmlichen, manuellen Entleerung über einem Ausguss 600, weil dabei ebenfalls die Effluentzulaufleitung 102 vom Effluentbeutel 400' getrennt werden muss.

Erst nachdem die Pumpe 131 für das Effluent steht, darf das Absperrelement 408, das z. B. ein Absperrhahn oder ein Abflussventil sein kann, geöffnet und die Pumpe 405 betrieben werden, um den Effluentbeutel 400' über die Effluentablaufleitung 403 in den Ausguss 600 zu entleeren.

Insbesondere falls die Pumpe 405 eine Impellerpumpe ist (aber nicht hierauf beschränkt), die nicht selbst ansaugend ist, kann es von Vorteil sein, wenn die Effluentablaufleitung 403 in Richtung Ausguss 600 ansteigend verlegt wird. Alternativ oder optional verläuft die Effluentablaufleitung 403 zwischen Absperrelement 408 und Pumpelement 405 abfallend verlegt, sodass Luftblasen aufsteigen und in Richtung Ausguss 600 oder alternativ Richtung Effluentbeutel 400' abgeführt werden können.

Bei diesem mit Bezug auf Fig. 6 erläuterten Verfahren achtet der Anwender aus Gründen der elektrischen Sicherheit (d. h. zum Unterschreiten der Grenzwerte für die Patientenableitströme) vorteilhafterweise darauf, dass die Pumpe 131 für das Effluent steht, bevor das Absperrelement 408 geöffnet und die Pumpe 405 betrieben wird.

Die Pumpe 405 kann als Pumpe, auf die in Fig. 4 die Bezugszeichen 141 und 405a hinweisen, ausgestaltet sein. Die Pumpe, gezeigt durch die Elemente mit den Bezugszeichen 141/405a, kann ausgestaltet sein, wie in der Deutschen Patentanmeldung, die für die Anmelderin der vorliegenden Anmeldung unter dem Aktenzeichen DE 102017122804.7 mit Anmeldetag 29. September 2017 beim Deutschen Patent- und Markenamt angemeldet wurde, offenbart ist. Es kann aber auch eine andere Pumpe verwendet werden.

Optional weist das Abflussschlauchsystem, das sich stromab des Effluentbeutels 400' anschließt, keinen Flussteiler auf. Das Abflussschlauchsystem kann hingegen eine Rollenpumpe aufweisen, etwa als Pumpe 405. Die Rollenpumpe kann optional mit genau einer Zuleitung und mit genau einer Ableitung verbunden sein. Damit kann die Pumpe den in sie hineinführenden Fluss offensichtlich nicht in mehrere Flüsse teilen.

Optional weist das Abflussschlauchsystem, das sich stromab des Effluentbeutels 400' und/oder stromab der Pumpe 405 anschließt, keinen Verbinder auf.

Optional weist das Abflussschlauchsystem, das sich stromab des Effluentbeutels 400' und/oder stromauf der Pumpe 405 anschließt, kein Element auf, das mit einer elektrischen Steuereinrichtung verbunden wäre, etwa in Form eines elektrisch verbundenen Verbinders.

Ein Vorteil dieser Variante des Verfahrens ist, dass ein herkömmlicher Effluentbeutel 400' verwendet werden kann. Lediglich die Effluentablaufleitung 403 stromabwärts des Effluentbeutels 400' mit der Pumpe 405 muss ergänzt werden. Dabei kann die Effluentablaufleitung 403 einige Merkmale in beliebiger Kombination, oder alle Merkmale, der Leitung 415 des in Fig. 4 gezeigten Ausführungsbeispiels (oder deren obenstehende Beschreibung) aufweisen, nämlich insbesondere einige oder alle Merkmale des in Fig. 4 gezeigten Leitungsabschnitts stromabwärts des Verbinders 411. Zur Vermeidung von Wiederholungen wird auf die Beschreibung der Fig. 4 verwiesen.

### Bezugszeichenliste

- 25: Zugabestelle für Heparin (optional)
- 29: venöse Blutkammer (optional)
- 31: Entlüftungseinrichtung

- 100: Blutbehandlungsvorrichtung
- 101: Blutpumpe
- 102: Dialysatablaufleitung, Effluentzulaufleitung
- 104: Dialysierflüssigkeitszulaufleitung
- 111: Pumpe für Substituat
- 121: Pumpe für Dialysierflüssigkeit
- 131: Pumpe für Dialysat oder Effluent in Effluentzulaufleitung
- 132: Konnektor
- 141: Pumpenantrieb für Pumpe 405 stromab des Drei-Wege-Hahns 401
- 143: Wiegehaken oder Wägehaken
- 150: Steuer- oder Regelvorrichtung

- 200: Quelle mit Dialysierflüssigkeit
- 201: Quelle mit Substituat, optional

- 300: extrakorporaler Blutkreislauf
- 301: erste Leitung (arterieller Leitungsabschnitt)
- 302: (erste) Schlauchklemme
- 303: Blutfilter oder Dialysator
- 303a: Dialysierflüssigkeitskammer
- 303b: Blutkammer
- 303c: semi-permeable Membran
- 305: zweite Leitung (venöser Leitungsabschnitt)
- 306: (zweite) Schlauchklemme
- 400: Effluentbeutel
- 400': Effluentbeutel
- 400a: Effluenteinlass- oder -auslassöffnung; Effluentöffnung
- 400'a: Effluenteinlassöffnung
- 400'b: Effluentauslassöffnung

- 401: Drei-Wege-Hahn, Umschalteinrichtung
- 401a: Verbinder
- 401b: Verbinder
- 401c: Verbinder
- 401d: Griffabschnitt

- 403: Effluentablaufleitung
- 405: Pumpe in Effluentablaufleitung
- 405a: Pumpkopf
- 407: Verbindungsleitung
- 408: Absperrelement, Absperrhahn, Abflussventil
- 409: Verbinder
- 411: Verbinder
- 415: Leitung
- 417: Verbinder
- 419: Rückschlagventil
- 421: Fixierungselement oder Befestigungsvorrichtung
- 423: Endstück

- 600: Ausguss

- H2: Beutelheizung mit Beutel (Dialysierflüssigkeit)
- H1: Beutelheizung mit Beutel (Substituat)

- PS1, PS2: arterieller Drucksensor (optional)
- PS3: Drucksensor (optional)
- PS4: Drucksensor zum Messen des Filtratdrucks

## Patentansprüche

1. Abflussschlauchsystem, aufweisend
- einen Effluentbeutel (400) zum Aufnehmen des bei einer Blutbehandlung anfallenden Effluents, wobei der Effluentbeutel (400) genau eine verschließbare Effluentöffnung (400a) oder Verbindung zu einem Äußeren des Effluentbeutels (400) aufweist, **gekennzeichnet durch**
- wenigstens eine zum Effluentbeutel (400) führende Effluentzulaufleitung (102), eine vom Effluentbeutel (400) wegführende Effluentablaufleitung (403), und
- eine Umschalteinrichtung (401), mittels welcher einander ausschließend entweder eine Fluidverbindung zwischen dem Inneren des Effluentbeutels (400) und der Effluentzulaufleitung (102) oder eine Fluidverbindung zwischen dem Effluentbeutel (400) und der Effluentablaufleitung (403) hergestellt werden kann.

2. Abflussschlauchsystem nach Anspruch 1, wobei die Umschalteinrichtung (401) ein Ventil, ein Drei-Wege-Hahn, ist oder aufweist.

3. Abflussschlauchsystem nach Anspruch 2, wobei die Umschalteinrichtung (401) aus Kunststoff oder Glas besteht oder wenigstens eines dieser Materialien aufweist.

4. Abflussschlauchsystem nach einem der vorangegangenen Ansprüche, wobei die Effluentablaufleitung (403) in Förderverbindung mit wenigstens einer Pumpe (405) und/oder einem Pumpenantrieb (141) einer Pumpe (405) steht.

5. Abflussschlauchsystem nach Anspruch 4, mit wenigstens einer Ladestation für eine Spannungsquelle für den Pumpenantrieb (141) der Pumpe (405).

6. Verfahren zum Entleeren eines Effluentbeutels (400), mit den Schritten:
- Bereitstellen eines Abflussschlauchsystems gemäß einem der Ansprüche 1 bis 5;
- Betätigen der Umschalteinrichtung (401) derart, dass eine Fluidverbindung zwischen dem Inneren des Effluentbeutels (400) und dem Inneren der verbundenen Effluentablaufleitung (403) hergestellt wird.

7. Verfahren nach Anspruch 6, wenn jener auf einen der Ansprüche 2 bis 5 rückbezogen ist, wobei der Pumpenantrieb (141), einer Blutbehandlungsvorrichtung (100) oder einer nicht zur Blutbehandlungsvorrichtung (100) zählenden Pumpvorrichtung manuell mit dem Pumpkopf (405a) verbunden wird.

8. Verfahren nach Anspruch 6 oder 7, wobei der Betrieb des Pumpenantriebs (141) manuell begonnen und/oder beendet wird.

9. Verfahren nach einem der Ansprüche 6 bis 8, mit dem Schritt:
- Betätigen der Umschalteinrichtung (401) derart, dass eine Fluidverbindung zwischen dem Inneren des Effluentbeutels (400) und dem Inneren der Effluentablaufleitung (403) unterbrochen wird.

10. Blutbehandlungsvorrichtung, verbunden mit einer Effluentzulaufleitung (102) und hierüber verbunden mit einem Abflussschlauchsystem gemäß einem der Ansprüche 1 bis 5, ausgestaltet als Hämodialysevorrichtung, Hämofiltrationsvorrichtung oder Hämodiafiltrationsvorrichtung.

11. Blutbehandlungsvorrichtung nach Anspruch 10, ausgestaltet als eine Vorrichtung für die chronische Nierenersatztherapie oder für die kontinuierliche Nierenersatztherapie (CRRT = continuous renal replacement therapy).

12. Blutbehandlungsvorrichtung nach einem der Ansprüche 10 bis 11, mit einer Ladestation für die Spannungsquelle für den Pumpenantrieb (141) der Pumpe (405).

## Claims

1. A discharge hose system comprising:
- an effluent bag (400) for receiving effluent produced during a blood treatment, wherein the effluent bag (400) has exactly one closable effluent opening (400a) or a connection to an exterior of the effluent bag (400), **characterized by**:
- at least one effluent inlet line (102) leading to the effluent bag (400), an effluent outlet line (403) leading away from the effluent bag (400), and
- a switching device (401), by means of which, mutually exclusively, either a fluid connection between the interior of the effluent bag (400) and the effluent inlet line (102) or a fluid connection between the effluent bag (400) and the effluent outlet line (403) can be established.

2. The discharge hose system according to claim 1, wherein the switching device (401) is, or comprises, a valve, a three-way-tap.

3. The discharge hose system according to claim 2, wherein the switching device (401) is made of plastic or glass or comprises at least one of these materials.

4. The discharge hose system according to anyone of the preceding claims, wherein the effluent outlet line (403) is in conveying connection with at least one pump (405) and/or a pump drive (141) of a pump (405).

5. The discharge hose system according to claim 4, comprising at least one charging point for a voltage source for the pump drive (141) of the pump (405).

6. A method of draining an effluent bag (400), comprising the steps of:
- providing a discharge hose system according to anyone of claims 1 to 5;
- operating the switching device (401) in such a way that a fluid connection between the interior of the effluent bag (400) and the interior of the connected effluent outlet line (403) is established.

7. The method according to claim 6, when referred back to anyone of claims 2 to 5, wherein the pump drive (141), of a blood treatment apparatus (100) or of a pump device not forming part of the blood treatment apparatus (100), is manually connected to the pump head (405a).

8. The method according to claim 6 or 7, wherein the operation of the pump drive (141) is manually started and/or stopped.

9. The method according to anyone of claims 6 to 8, comprising the step of:
- operating the switching device (401) in such a way that a fluid connection between the interior of the effluent bag (400) and the interior of the effluent outlet line (403) is interrupted.

10. A blood treatment apparatus, connected to an effluent inlet line (102) and connected via this to a discharge hose system according to anyone of claims 1 to 5, designed as a hemodalysis apparatus, a hemofiltration apparatus or a hemodiafiltration apparatus.

11. The blood treatment apparatus according to claim 10, designed as an apparatus for chronic renal replacement therapy or for continuous renal replacement therapy (CRRT = continuous renal replacement therapy).

12. The blood treatment apparatus according to anyone of claims 10 to 11, comprising a charging point for the voltage source for the pump drive (141) of the pump (405).

## Revendications

1. Un système de tuyaux d'évacuation comprenant:
- une poche à effluent (400) destinée à recevoir l'effluent produit au cours d'un traitement du sang, la poche à effluent (400) présentant précisément une ouverture d'effluent (400a) refermable ou une connexion à un extérieur de la poche à effluent (400),
**caractérisé par**:
- au moins une conduite d'entrée d'effluent (102) menant à la poche à effluent (400), une conduite d'évacuation d'effluent (403) s'éloignant de la poche à effluent (400), et
- un dispositif de commutation (401), au moyen duquel,
de manière mutuellement exclusive, soit une liaison fluidique entre l'intérieur de la poche à effluent (400) et la conduite d'entrée d'effluent (102), soit une liaison fluidique entre la poche à effluent (400) et la conduite de sortie d'effluent (403) peut être établie.

2. Le système de tuyaux d'évacuation selon la première revendication, où le dispositif de commutation (401) est, ou comprend, une vanne, un robinet à trois voies.

3. Le système de tuyaux d'évacuation selon la revendication 2, où le dispositif de commutation (401) est constitué de plastique ou de verre, ou comprend au moins un de ces matériaux.

4. Le système de tuyaux d'évacuation selon l'une quelconque des revendications précédentes, où la conduite d'évacuation d'effluent (403) est en liaison d'acheminement avec au moins une pompe (405) et/ou un entraînement de pompe (141) d'une pompe (405).

5. Le système de tuyaux d'évacuation selon la revendication 4, comprenant au moins une station de charge pour une source de tension pour l'entraînement de pompe (141) de la pompe (405).

6. Un procédé de vidange d'une poche à effluent (400) comprenant les étapes suivantes:
- la fourniture d'un système de tuyaux d'évacuation selon l'une quelconque des revendications 1 à 5;
- l'actionnement du dispositif de commutation (401) de telle sorte qu'une liaison fluidique soit établie entre l'intérieur de la poche à effluent (400) et l'intérieur de la conduite d'évacuation d'effluent (403) reliée.

7. Le procédé selon la revendication 6, lorsqu'il se réfère à l'une quelconque des revendications 2 à 5, où l'entraînement de pompe (141), d'un appareil de traitement du sang (100) ou d'un dispositif de pompage ne faisant pas partie de l'appareil de traitement du sang (100), est relié manuellement à la tête de pompe (405a).

8. Le procédé selon la revendication 6 ou 7, où l'opération de l'entraînement de pompe (141) est démarrée ou terminée manuellement.

9. Le procédé selon l'une quelconque des revendications 6 à 8, comprenant l'étape suivante:
- l'actionnement du dispositif de commutation (401) de telle sorte qu'une liaison fluidique entre l'intérieur de la poche à effluent (400) et l'intérieur de la conduite d'évacuation d'effluent (403) soit interrompue.

10. Un appareil de traitement du sang relié à la conduite d'entrée d'effluent (102) et relié par ce biais à un système de tuyaux d'évacuation selon l'une quelconque des revendications 1 à 5, conçu comme un appareil d'hémodialyse, un appareil d'hémofiltration ou un appareil d'hémodiafiltration.

11. L'appareil de traitement du sang selon la revendication 10, conçu comme un appareil pour la thérapie de remplacement rénal chronique ou pour la thérapie de remplacement rénal continue (CRRT = continuous renal replacement therapy).

12. L'appareil de traitement du sang selon l'une quelconque des revendications 10 à 11, comprenant une station de charge pour la source de tension pour l'entraînement de pompe (141) de la pompe (405).
